# FASCICULE DE BREVET EUROPEEN

(11) **EP 3 968 891 B1**
(45) Date de publication et mention de la délivrance du brevet: **27.12.2023**
(21) Numéro de dépôt: 20740706.5
(22) Date de dépôt: 12.05.2020
(51) Int. Cl.: A61B 17/86, A61C 8/00

(54) **DISPOSITIF POUR LE MAINTIEN ET LA LIBÉRATION D'UN OBJET ET PROCÉDÉS CORRESPONDANTS**
VORRICHTUNG ZUM HALTEN UND FREIGEBEN EINES OBJEKTS UND ZUGEHÖRIGE VERFAHREN
DEVICE FOR HOLDING AND RELEASING AN OBJECT AND CORRESPONDING METHODS

(30) Priorité: 14.05.2019 FR 1905009; 14.05.2019 US 201962847695 P
(43) Date de publication de la demande: 23.03.2022
(73) Titulaire: Selenium Medical, 17000 La Rochelle (FR)
(72) Inventeur: RICHART, Olivier, 17580 Le Bois Plage en Re (FR)
(74) Mandataire: Ipsilon
(86) Numéro de dépôt international: PCT/FR2020/050784
(87) Numéro de publication internationale: WO 2020/229770

(56) Documents cités:
- EP-A1- 2 995 271
- WO-A1-02/30315
- KR-A- 20170 116 673

## Description

### DOMAINE DE L'INVENTION

La présente invention concerne de manière générale un dispositif permettant de maintenir un objet, tel qu'un implant médical, et de libérer ledit objet par rapport à ce dispositif.

### ART ANTERIEUR

Le document KR20170116673A décrit un dispositif à lame de coupe muni d'un capuchon de protection de la lame de coupe. Le document WO0230315 décrit un emballage pour implant comprenant un corps et un système coulissant permettant de sortir l'implant par rapport au corps.

On connaît de l'état de la technique et notamment de la demande internationale WO2019030451A2 et de la demande de brevet français déposée sous le numéro FR1854199, non encore publiée avant la date de priorité de la présente demande de brevet, des dispositifs de maintien d'objet, encore appelés préhenseurs.

Le dispositif présenté dans la demande de brevet français FR1854199 permet en particulier de maintenir à l'aide d'un système de crochets, un implant de type vis qui présente une tête apte à venir en butée contre le système de crochets pour empêcher l'implant de tomber au fond du corps du dispositif.

Cependant pour des implants dépourvus de tête apte à venir en butée avec le dispositif, il existe un risque que l'implant ne glisse au fond du corps du dispositif.

Il est ainsi souhaitable de pouvoir proposer un autre dispositif de maintien d'objet pour lequel l'objet peut être maintenu de manière fiable dans une position donnée, tout en permettant de le libérer aisément du dispositif.

### RESUME DE L'INVENTION

A cet effet, il est proposé un dispositif pour le maintien et la libération d'un objet médical selon la revendication 1.

Le dispositif de maintien et de libération d'objet permet ainsi d'offrir une butée basse vis-à-vis de l'objet, la position de cette butée basse étant réglable pour être adaptée à la longueur de l'objet, tout en empêchant un retour en arrière de cette butée basse pour éviter que l'objet ne tombe dans le corps principal, ce qui compliquerait son extraction.

Lors de la production du dispositif, c'est-à-dire de la mise en place de l'objet dans le dispositif, au moins une partie de l'objet est introduite dans le corps par la sortie du corps qui sert alors également de passage d'introduction de l'objet dans le corps. Une autre partie de l'objet, par exemple une deuxième pièce distincte d'une première pièce à introduire dans le corps par la sortie du corps, peut être logée en partie dans le dispositif de butée.

L'accès à la sortie du corps principal depuis l'extérieur du corps est ensuite obturé ou entravé par une mise en position de fermeture du capot. Par ailleurs, préalablement au passage en position de fermeture du capot, un élément de scellage peut être appliqué sur le corps au niveau de la sortie dudit corps.

Une fois l'objet introduit dans le corps principal, le dispositif de butée est ensuite remonté vers l'objet pour amener ledit objet en butée haute contre le capot, le dispositif de butée formant butée basse vis-à-vis de l'objet.

Le dispositif peut aussi comporter une ou plusieurs des caractéristiques suivantes prises dans toute combinaison techniquement admissible.

Selon une caractéristique particulière, le dispositif comprend un système de crémaillère configuré pour permettre ledit déplacement du dispositif de butée en direction de la sortie dudit corps principal, avec blocage en sens inverse.

Selon une caractéristique particulière, le dispositif de butée est configuré pour être déplaçable par rapport au corps principal sur une distance permettant d'amener au moins une partie de l'objet en saillie du corps principal.

Selon une caractéristique particulière, le dispositif de butée présente un logement apte à recevoir une partie de l'objet.

Ce logement du dispositif de butée permet en particulier, en entourant ladite partie de l'objet, de maintenir l'orientation axiale de ladite partie de l'objet.

Selon une caractéristique particulière, le logement du corps principal comprend une bague à travers laquelle au moins une partie de l'objet est apte à s'étendre, ladite bague étant réalisée en un matériau différent de celui du corps principal.

Cela permet à l'objet d'être en contact avec un matériau compatible avec son propre matériau, par exemple le titane, sans avoir à réaliser l'ensemble du corps principal du dispositif dans ce matériau qui peut être onéreux.

Selon un aspect particulier, la bague présente un diamètre intérieur légèrement supérieur au diamètre extérieur de l'objet qui traverse cette bague pour permettre un guidage axial sans coincement de l'objet.

Ainsi, il peut être prévu qu'en position ouverte du capot et en l'absence ou à l'état retiré d'un élément de scellage de la sortie du corps, au moins la partie de l'objet qui traverse la bague peut être sortie du corps simplement en inclinant la sortie du corps du dispositif vers le bas.

Selon un aspect particulier, la bague s'étend en retrait de l'extrémité supérieure du corps principal. Avantageusement, ladite extrémité supérieure du corps principal délimite un passage qui peut être scellé.

Selon un aspect particulier, le logement de l'objet ménagé dans le corps principal présente une partie entre la bague et le dispositif de butée, qui reste ouverte sur l'intérieur du corps principal.

Selon un aspect particulier, le logement de l'objet comprend une jupe qui s'étend au moins sous la bague, et le dispositif de butée comprend une partie d'extrémité formant piston apte à s'étendre de manière étanche à l'intérieur de la jupe.

Le dispositif de butée présente ainsi une partie formant piston configurée pour être déplacée à l'intérieur de la jupe, de manière étanche par rapport à la jupe en direction de la sortie du corps principal, sans recul possible.

Selon un aspect particulier, la jupe contient un fluide, de préférence un liquide, ledit objet étant logé dans une chambre définie au moins par ladite jupe et ladite bague, ladite chambre étant fermée en partie basse par le piston du dispositif de butée et en partie haute par un système de scellage.

Selon un aspect particulier, la sortie du corps principal est scellée par un système de scellage retirable.

Selon un aspect particulier, le système de scellage est couplé avec le capot de telle sorte que le passage du capot de la position de fermeture à la position d'ouverture entraîne le retrait du système de scellage.

Selon un aspect particulier, le dispositif comprend un système de stabilisation configuré pour stabiliser le capot en position d'ouverture et en position de fermeture.

Selon un aspect particulier, le capot est configuré pour, en position d'ouverture, rester couplé au corps principal en formant un pied permettant de disposer le corps principal en appui par ce pied sur une surface, tandis que la sortie dudit corps principal est surélevée par rapport à cette surface.

Selon un aspect particulier, le capot comprend un pion de centrage apte à venir en contact avec une extrémité de l'objet en position de fermeture du capot.

Selon un aspect particulier, ledit dispositif comprend ledit objet médical.

Selon un aspect particulier, ledit objet comprend deux pièces distinctes.

Selon un aspect particulier, ledit objet étant de type dentaire, ledit objet comprend une vis-implant et une vis de cicatrisation.

En particulier, la vis-implant se présente sous la forme d'un corps allongé fileté avec une extrémité creuse, mais dépourvue d'une tête dont la largeur serait supérieure au diamètre du corps de vis.

L'invention concerne également un procédé de production d'un dispositif de maintien et de libération d'un objet médical, le dispositif étant tel que présenté ci-dessus, le procédé comprenant les étapes suivantes :
- le capot étant en position d'ouverture, introduction d'au moins une partie de l'objet dans le corps principal par la sortie dudit corps principal ;
- déplacement du dispositif de butée en direction de la sortie dudit corps principal, pour empêcher ou limiter le déplacement de l'objet dans le corps principal, le recul du dispositif de butée étant empêché.

Selon un aspect particulier, ladite au moins une partie de l'objet introduite dans le corps principal par la sortie étant une première pièce, ledit procédé comprend, avant ladite étape de déplacement du dispositif de butée, l'étape d'introduction dans le dispositif de butée d'une deuxième pièce, ladite étape de déplacement du dispositif de butée en direction de la sortie dudit corps principal amenant ladite deuxième pièce en contact ou à proximité de la première pièce, le recul du dispositif de butée étant empêché. La deuxième pièce peut être introduite avant la première pièce ou inversement.

Selon un aspect particulier, ledit procédé comprend l'étape de remplissage en fluide ou en liquide d'une chambre du corps principal dans laquelle loge au moins une partie dudit objet.

Selon un aspect particulier, ledit procédé comprend une étape de scellage de la sortie du corps principal.

Selon un aspect particulier, ledit procédé comprend une étape de fermeture du capot, avant ou après le déplacement du dispositif de butée.

L'invention concerne également un procédé de libération d'un objet médical maintenu dans un dispositif tel que présenté ci-dessus, ledit procédé comprenant les étapes suivantes :
- faire passer le capot en position d'ouverture,
- extraire au moins une partie de l'objet par la sortie du corps principal à l'aide d'un outil coopérant avec ladite au moins une partie de l'objet.

Selon un aspect particulier, ledit procédé comprend les étapes de :
- pousser le dispositif de butée en direction de la sortie du corps principal pour amener une autre partie de l'objet à proximité de la sortie du corps principal, et
- extraire ladite autre partie de l'objet par la sortie du corps principal à l'aide d'un outil coopérant avec ladite autre partie de l'objet.

L'invention concerne également un procédé de libération d'un objet médical maintenu dans un dispositif tel que présenté ci-dessus, ledit procédé comprenant les étapes suivantes :
- faire passer le capot en position d'ouverture,
- pousser le dispositif de butée pour amener une partie de l'objet en saillie de la sortie du corps principal,
- extraire ladite partie de l'objet qui s'étend en saillie de la sortie du corps principal,
- extraire une autre partie de l'objet à travers la sortie du corps principal à l'aide d'un outil coopérant avec ladite autre partie de l'objet.

### BREVE DESCRIPTION DES DESSINS

D'autres caractéristiques et avantages de l'invention ressortiront encore de la description qui suit, laquelle est purement illustrative et non limitative et doit être lue en regard des dessins annexés, sur lesquels :
[Fig. 1] la figure 1 est une vue en perspective avant d'un dispositif conforme à un mode de réalisation de l'invention ;
[Fig. 2] la figure 2 est une vue en perspective du côté intérieur du coulisseau du dispositif de la figure 1 ;
[Fig. 3] la figure 3 est une vue en perspective du corps du dispositif de la figure 1, côté face avant du corps ;
[Fig. 4] la figure 4 reprend la vue de la figure 1, avec une vue en coupe axiale de la partie supérieure du corps du dispositif ;
[Fig. 5] la figure 5 reprend la vue de la figure 1, avec vue en coupe axiale de la partie supérieure du coulisseau ;
[Fig. 6] la figure 6 reprend la vue de la figure 1, avec vue en arraché d'une partie du coulisseau laissant apparaitre le mécanisme de coulissement du coulisseau sur le corps du dispositif ;
[Fig. 7] la figure 7 est une vue en perspective du dispositif de la figure 1, à l'état ouvert du capot et en position haute du coulisseau de sorte que la vis implant s'étend en partie en saillie du corps du dispositif ;
[Fig. 8] la figure 8 est une vue du capot d'un dispositif conforme à un mode de réalisation de l'invention ;
[Fig. 9] la figure 9 reprends le dispositif de la figure 8 avec vue en coupe de la partie supérieure du corps du dispositif et de la partie supérieure du coulisseau ;
[Fig. 10] la figure 10 est une vue en perspective de dessus du dispositif de la figure 9 à l'état retiré de l'implant, la vis de cicatrisation étant encore présente dans la partie supérieure du coulisseau ;
[Fig. 11] la figure 11 est une vue en perspective arrière du dispositif conforme à un mode de réalisation de selon l'invention, permettant d'apercevoir à travers la fenêtre ménagée dans la face arrière du dispositif une partie de l'implant et de la vis de cicatrisation ;
[Fig. 12] la figure 12 est une vue en perspective d'un dispositif selon un autre mode de réalisation ;
[Fig. 13] la figure 13 est une vue en perspective d'un dispositif identique ou similaire à celui de la figure 12, en cours d'ouverture du capot ;
[Fig. 14] la figure 14 est une vue en perspective du dispositif de la figure 13, avec vue en coupe de la partie supérieure du corps du dispositif, l'élément de scellage de la sortie du corps n'étant pas représenté, le coulisseau étant en position basse et munie à son extrémité haute d'un élément formant piston qui s'étend dans le logement tubulaire ;
[Fig. 15] la figure 15 est une vue du dispositif de la figure 14, à l'état ouvert du capot et en position remontée du coulisseau, de sorte que l'implant, poussé par le piston du coulisseau, s'étend en saillie de la bague présente dans la partie supérieure du corps ;
[Fig. 16] la figure 16 est une vue partielle d'un coulisseau selon un autre mode de réalisation conforme à l'invention, l'extrémité supérieure du coulisseau formant piston étant représentée coupée axialement ;
[Fig. 17] la figure 17 est une vue partielle d'un coulisseau selon un autre mode de réalisation conforme à l'invention, l'extrémité supérieure du coulisseau formant piston étant représentée coupée axialement ;
[Fig. 18] la figure 18 est une vue partielle d'un coulisseau selon un autre mode de réalisation conforme à l'invention, l'extrémité supérieure du coulisseau formant piston étant représentée coupée axialement ;
[Fig. 19] la figure 19 est une vue de côté d'un dispositif conforme à un mode de réalisation de l'invention, en position d'ouverture du capot, l'implant étant sorti du corps du dispositif à l'aide d'un outil de saisie adapté ;
[Fig. 20] la figure 20 est une vue en perspective d'un dispositif conforme à un mode de réalisation de l'invention, en position d'ouverture du capot, l'implant étant logé dans le corps du dispositif avec une extrémité légèrement en saillie de la bague ;

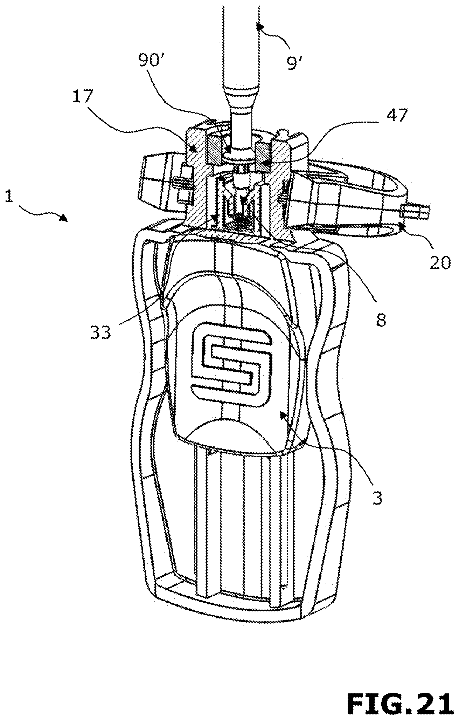

[Fig. 21] la figure 21 est une vue en perspective d'un dispositif conforme à un mode de réalisation de l'invention, en position d'ouverture du capot, le coulisseau étant poussé en position haute pour amener la vis de cicatrisation à proximité de la sortie du corps, un outil étant introduit à travers la bague pour venir chercher la vis de cicatrisation et l'extraire du corps du dispositif.

### DESCRIPTION DETAILLEE

Le concept de l'invention est décrit plus complètement ci-après avec référence aux dessins joints, sur lesquels des modes de réalisation du concept de l'invention sont montrés. Sur les dessins, la taille et les tailles relatives des éléments peuvent être exagérées à des fins de clarté. Des numéros similaires font référence à des éléments similaires sur tous les dessins. Cependant, ce concept de l'invention peut être mis en oeuvre sous de nombreuses formes différentes et ne devrait pas être interprété comme étant limité aux modes de réalisation exposés ici. Au lieu de cela, ces modes de réalisation sont proposés de sorte que cette description soit complète, et communiquent l'étendue du concept de l'invention aux hommes du métier. Les modes de réalisation qui suivent sont examinés, par souci de simplification, en relation avec la terminologie et la structure d'un dispositif permettant de maintenir et de libérer un objet, tel qu'un implant médical.

En référence aux figures, on a représenté un dispositif 1 pour le maintien et la libération d'un objet. Dans les exemples illustrés aux figures, ledit objet est de type médical, en particulier dentaire.

Comme expliqué ci-après au travers de différents modes de réalisation du dispositif, ledit objet peut comprendre une ou plusieurs pièces, par exemple un implant médical et une pièce accessoire. Le dispositif 1 peut encore être appelé préhenseur. Un tel dispositif 1 peut être utilisé par un opérateur pour manipuler un objet par l'intermédiaire du dispositif dans de bonnes conditions d'asepsie notamment en utilisant un outil adapté pour extraire l'objet.

Le dispositif 1 peut être logé dans un emballage ou double-emballages tel que décrit dans la demande internationale WO2014188142 ou la demande internationale WO2018078242.

Le dispositif 1 comprend un corps 10 principal configuré pour permettre de loger au moins une partie dudit objet et un capot 20 monté mobile entre une position de fermeture dans laquelle il empêche une sortie dudit objet par rapport au corps et une position d'ouverture dans laquelle il permet la sortie dudit objet ou permet l'accès audit objet pour l'extraire du corps 10. Selon un aspect particulier, la position d'ouverture permet aussi d'introduire ou de réintroduire au moins une partie dudit objet dans le corps 10.

Le corps 10 du dispositif 1 présente ainsi une sortie par laquelle, en position d'ouverture du capot, ledit objet peut être sorti.

Le dispositif 1 comprend aussi un dispositif de butée 3 qui permet de mettre en butée basse l'objet introduit dans le corps du dispositif, en particulier pour éviter qu'il ne tombe au fond du corps 10 du dispositif 1.

Le dispositif 1 est configuré pour permettre de déplacer le dispositif de butée 3 dans la direction de l'objet (c'est-à-dire en direction de la sortie du corps), et pour empêcher un déplacement du dispositif de butée 3 dans le sens d'un éloignement par rapport à l'objet (c'est-à-dire empêcher un déplacement du dispositif de butée 3 dans la direction opposée à la sortie du corps).

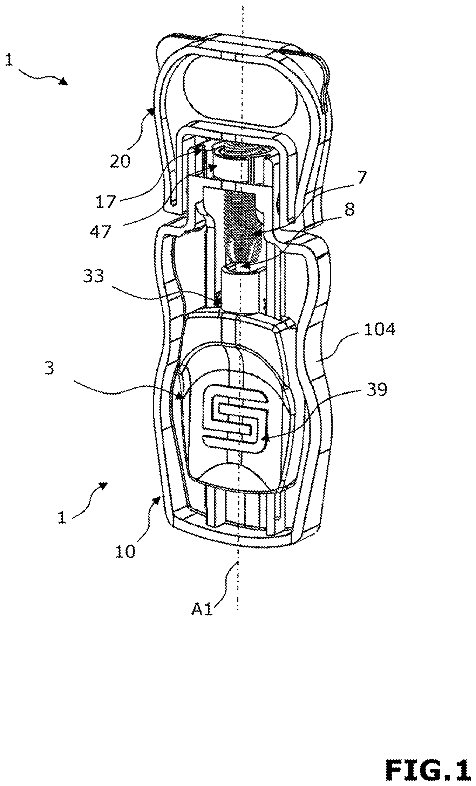

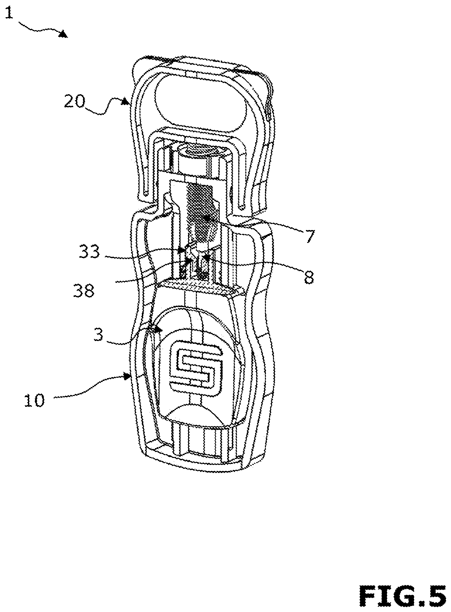

Dans les exemples illustrés aux figures, le dispositif de butée 3 est formé par un coulisseau 3. Le coulisseau 3 est déplaçable le long du corps 10 pour venir en contact de l'objet. On peut prévoir que le coulisseau 3 vienne simplement en contact avec l'objet, mais on peut aussi prévoir comme dans l'exemple illustré aux figures 1 à 11, qu'une partie de ce coulisseau présente un logement permettant de recevoir la partie inférieure de l'objet. Le coulisseau 3 permet de venir au contact de l'objet, notamment pour maintenir l'objet en position dans le corps et éviter qu'il ne tombe au fond du corps. Le coulisseau 3 forme ainsi une butée basse réglable en position dans le sens montant, c'est-à-dire en direction de la sortie du corps, pour s'adapter à la longueur de l'objet introduit dans le corps du dispositif.

Selon un aspect particulier, le coulisseau 3 est déplaçable sur une distance permettant à l'opérateur de pousser l'objet en partie en dehors du corps 10, par déplacement du coulisseau 3 en direction de l'objet, lorsque le capot 20 est en position ouverte. Comme détaillé ci-après, cette fonction est particulièrement utile lorsque l'objet comprend deux pièces dont une portée par le coulisseau 3. Après avoir extrait la première pièce par la sortie, la deuxième pièce précédemment située entre le coulisseau et la sortie du corps peut se retrouver à une distance trop importante de la sortie du corps pour pouvoir être saisie aisément, en particulier lorsque l'outil de saisie comprend un épaulement l'empêchant d'être engagé profondément dans le corps du dispositif ou risquant de gêner la sortie de l'outil. Le fait de pouvoir remonter encore le coulisseau et donc de rapprocher la deuxième pièce de la sortie du corps facilite l'opération de sortie.

Selon un aspect particulier, le déplacement du coulisseau 3 vers la sortie du corps 10 est limité par un épaulement du corps 10 ménagé au niveau de la partie supérieure dudit corps 10.

### Objet logé dans le corps du dispositif

Comme rappelé ci-dessus et comme détaillé ci-après au travers de différents modes de réalisation, ledit objet peut comprendre plusieurs pièces, par exemple un implant dentaire et une vis de cicatrisation.

On peut prévoir que l'objet comprend au moins une vis implant 7 (implant dentaire), c'est-à-dire un corps allongé fileté, qui traverse de préférence une bague 47 comme expliqué ci-après, et comprend éventuellement une pièce supplémentaire 8, telle qu'une vis de cicatrisation, de préférence logée en partie dans le coulisseau, qui s'étend entre la vis implant 7 et le coulisseau 3. L'implant dentaire 7 se présente sous la forme d'un corps fileté dont l'extrémité supérieure située du côté de la sortie du corps est creuse avec une forme intérieure adaptée à coopérer avec un outil 9 de saisie correspondant.

Dans les modes de réalisation des figures 1 à 11, l'objet comprend ainsi un implant dentaire 7 et une vis de cicatrisation 8.

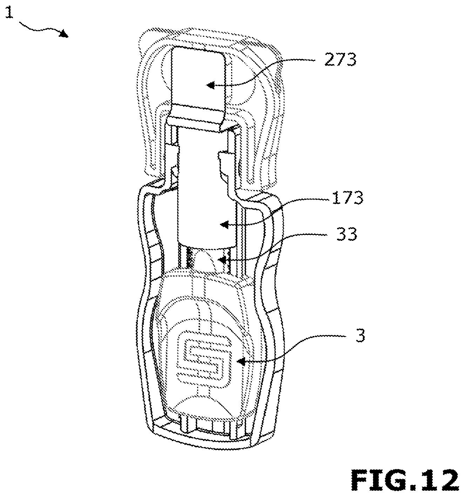

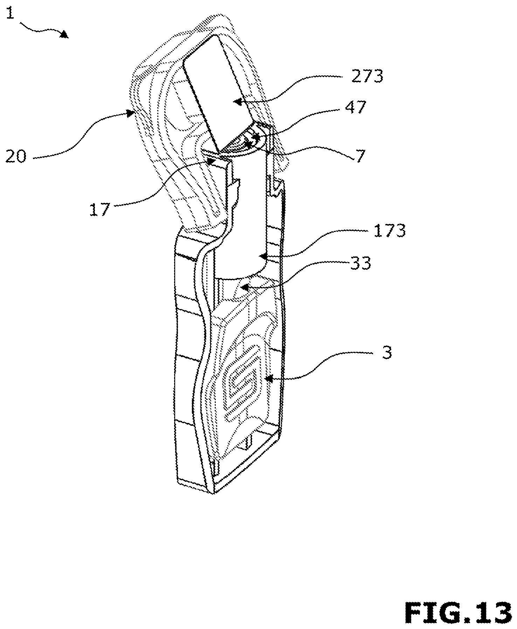

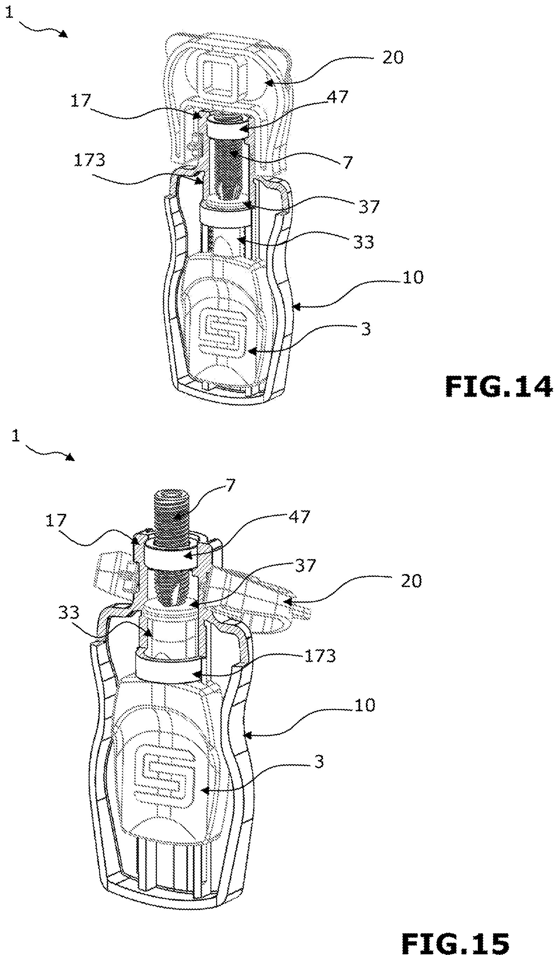

Dans les exemples des figures 12 à 15, seul l'implant 7 est représenté. On peut prévoir que l'implant 7 soit seul ou associé à une vis de cicatrisation comme pour les modes de réalisation des figures 1 à 11.

Avantageusement, dans ces modes de réalisation des figures 12 à 15, l'implant dentaire 7 baigne dans un fluide, de préférence un liquide, contenu dans une chambre formée par une jupe 173 (par exemple formée par un tube) en partie supérieure du corps 10 du dispositif. On considère comme partie supérieure du corps 10, ou partie supérieure du coulisseau 3, la partie du corps 10 ou la partie du coulisseau 3 qui est située du côté du capot 20 en position de fermeture de celui-ci.

Avant ouverture du capot 20 et/ou avant poussée sur le coulisseau 3 par l'opérateur, l'extrémité inférieure de la jupe 173 est fermée par le piston 37 du coulisseau 3, et l'extrémité supérieure de la jupe 173 est fermée par un élément de scellage 273, tel qu'une bande ou feuille pelable, qui peut être thermocollante, par exemple en matériau synthétique non-tissé fabriquée à partir de fibres de polyéthylène, usuellement vendue sous la marque déposée TYVEK. L'élément de scellage 273 peut aussi être en aluminium. Avantageusement, à l'état initial, c'est-à-dire en position fermée du capot 20 et avant déplacement de l'implant 7 dans le sens d'une sortie, ledit implant 7 ne fait pas saillie de l'extrémité supérieure du corps 10 en direction de l'extérieur du corps, mais se situe juste en dessous de ce niveau d'extrémité du corps destiné à être recouvert par l'élément de scellage.

Comme détaillé ci-après, le dispositif 1 dans lequel est logé la vis implant 7, et éventuellement la vis 8 de cicatrisation, permet aussi de libérer la vis implant 7 qui peut alors être extraite du dispositif par exemple à l'aide d'un outil 9 adapté, par exemple un outil de type tournevis, présentant un embout apte à coopérer avec l'intérieur de la tête de la vis implant 7, pour coupler la vis implant 7 à l'outil 9.

Comme détaillé ci-après, la vis implant 7 est repositionnable dans son logement. En particulier, dans le cas où un opérateur tel qu'un chirurgien a besoin de mettre en attente la vis implant pour effectuer une autre tâche.

### Corps principal

Le corps 10 principal comprend un passage de sortie. Le passage de sortie est situé à une extrémité, appelée extrémité supérieure, dudit corps 10 principal pour permettre la sortie de la vis implant 7 et de la vis 8 de cicatrisation selon un axe A1 de sortie. Comme détaillé ci-après, ce passage de sortie est aussi utilisé pour introduire l'implant 7 dans le corps du dispositif.

Le passage par lequel l'implant 7 est introduit dans le corps 10 et par lequel il peut être sorti dudit corps 10 peut être considéré comme un passage traversant qui débouche dans le corps 10 en regard du coulisseau 3, et qui dans certains modes de réalisation peut être fermé en partie inférieure par ledit coulisseau 3, en particulier quand le passage comprend une jupe 173 comme dans les modes de réalisation des figures 12 à 15 et que cette jupe forme avec le coulisseau 3 une chambre dans laquelle baigne l'implant 7.

Comme rappelé ci-dessus l'autre extrémité de la chambre qui contient l'implant 7 est de préférence fermée par un élément, tel qu'une feuille ou bande de scellage, retirable en même temps ou après ouverture du capot 20.

Dans l'exemple illustré aux figures, le corps principal 10 est de formée allongée selon l'axe A1 de sortie de l'implant 7 et de la vis 8.

Le corps 10 présente une paroi de fond 100. Avantageusement, l'axe de sortie A1 s'étend entre la paroi de fond 100 et la face avant ouverte dudit corps 10 qui est opposée à ladite paroi de fond 100.

La paroi de fond 100 présente une face intérieure 101 munie de nervures 13 le long desquelles le coulisseau 3 présenté ci-après est configuré pour pouvoir coulisser en direction de la partie supérieure 17 du corps 10.

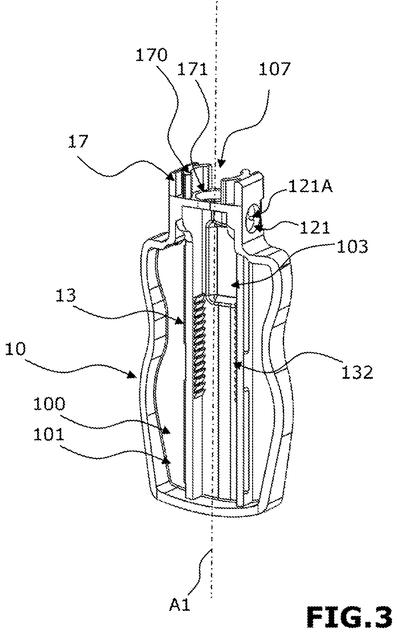

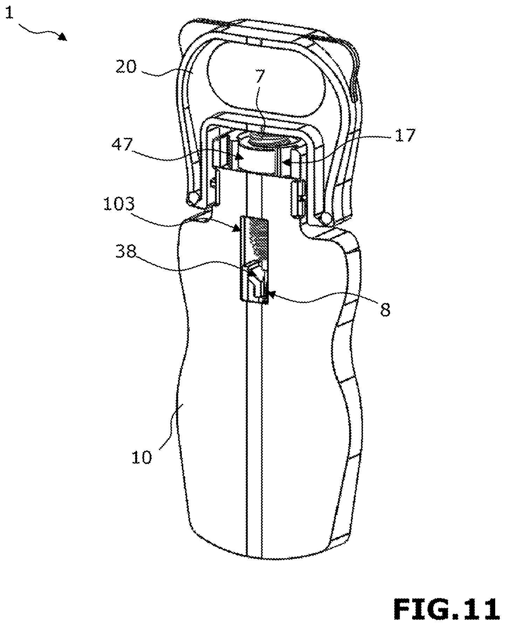

Comme visible à la figure 3 et à la figure 11, une fenêtre 103 peut être ménagée à travers la paroi de fond 100 pour permettre de visualiser la présence de l'objet depuis la face externe 102 de ladite paroi de fond 100. L'implant 7 s'étend du côté de la face interne de la paroi de fond en regard de ladite fenêtre. On peut prévoir que la fenêtre 103 ne soit pas présente.

Avantageusement, le corps du dispositif, et/ou le capot et/ou le coulisseau est ou sont transparents ou translucides.

Le corps 10 du dispositif présente une paroi périphérique 104 qui s'étend transversalement, de préférence orthogonalement, à la paroi de fond 101, par laquelle l'opérateur peut saisir le corps du dispositif. Avantageusement, les côtés latéraux opposés dudit corps 10 présentent chacun une partie courbe en renfoncement pour faciliter la saisie dudit corps du dispositif par l'opérateur.

### Système de coulissement du coulisseau par rapport au corps

Comme évoqué ci-avant, le dispositif 1 de maintien et de libération d'implant comprend un système de coulissement du coulisseau 3 par rapport au corps 10 du dispositif 1.

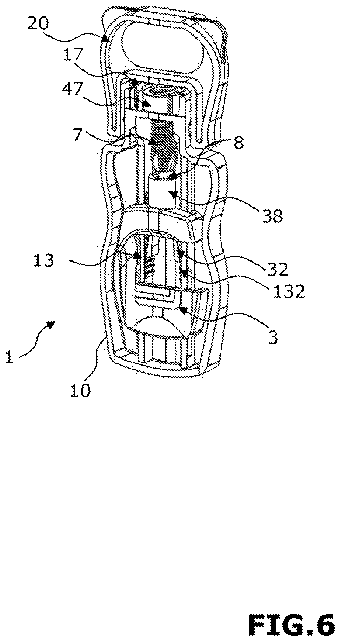

Comme illustré aux figures 3 et 6, le système de coulissement comprend deux rail 13 parallèles, de préférence ménagés sur la paroi de fond 100 du côté intérieur du corps. A cet effet le dispositif de butée 3 est muni de rainures 31 (ou gorges) coopérant avec lesdits rails. Bien entendu, les rails 13 (nervures) pourraient être portés par le coulisseau 3 et les rainures ou gorges pourraient être ménagées dans le corps 10.

Dans l'exemple illustré aux figures, le corps 10 présente un système de crantage, tel qu'un système à crémaillère, configuré pour permettre un déplacement du coulisseau 3 vers l'extrémité haute du corps 10 au niveau de laquelle se situe l'objet, mais empêchant un déplacement du coulisseau 3 en direction opposée. Dans l'exemple illustré aux figures, le système à crémaillère comprend pour chaque nervure 13 une crémaillère 132 solidaire de ladite nervure 13.

En particulier, dans la vue de la figure 6, une partie du coulisseau 3 a été échancrée dans sa zone milieu pour montrer le système de coulissement entre le coulisseau 3 et le corps 10 du dispositif 1.

Le coulisseau 3 est muni de dents 32 aptes à coopérer avec les dents de la crémaillère. Les dents de la crémaillère 132 du corps 10 et les dents 32 du coulisseau 3 sont orientées de manière à permette un déplacement du coulisseau 3 uniquement vers l'extrémité supérieure du corps du dispositif.

### Partie supérieure du corps du dispositif

Selon des modes de réalisation, la partie supérieure 17 du corps 10 du dispositif 1 au niveau de laquelle se situe le passage d'entrée/sortie de l'implant 7 comprend une bague 47 (encore appelée collerette) rapportée dans un logement correspondant ménagé dans la partie supérieure 17 du corps 10 du dispositif 1.

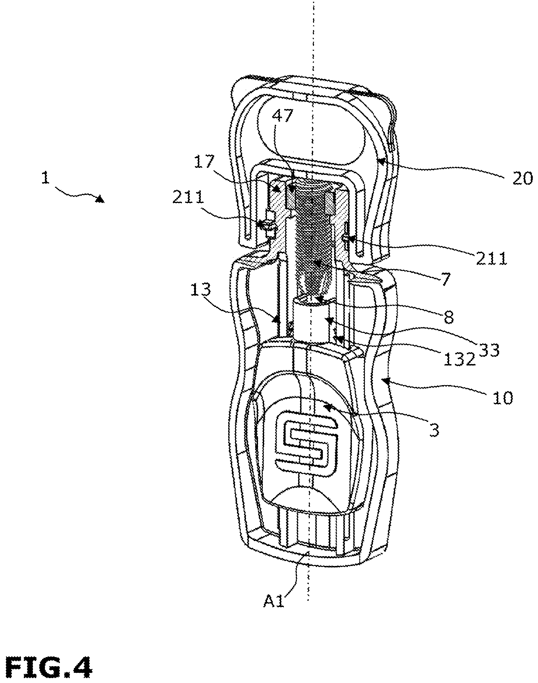

La figure 4 montre en coupe axiale la partie supérieure 17 du corps du dispositif, ce qui fait apparaître le système de pivotement du capot par rapport au corps, ainsi que la bague 47.

La bague 47 est réalisée dans un matériau différent de celui du corps du dispositif 1. Avantageusement, la bague 47 est réalisée dans un matériau de la même famille ou identique à celui utilisé pour l'implant 7. Avantageusement, l'implant 7 et la pièce 8 sont aussi réalisés dans un matériau de la même famille ou identique. Ainsi on peut prévoir que le corps 10 du dispositif soit réalisé en plastique, tandis que la bague 47 est réalisée en titane, l'implant 7 étant en titane.

Une telle bague permet de guider l'implant tout en évitant que l'implant soit en contact avec un matériau qui ne serait pas compatible avec celui de l'implant.

Dans les exemples illustrés aux figures, la partie supérieure 17 comprend en particulier un siège 171 configuré pour recevoir la bague 47. Une paroi murale 170 entoure au moins partiellement le siège 171 pour délimiter le logement de réception de la bague 47. La bague 47 est de préférence montée à force dans le logement de réception correspondant du corps 10.

Le siège 171 présente un passage traversant aligné avec le passage traversant de la bague 47. L'implant 7 est ainsi apte à s'étendre à travers la bague 47 et à travers le passage traversant du siège 171.

Autrement dit, le corps 10 présente ainsi un logement permettant de contenir au moins une partie de l'implant 7. Les passages traversants de la bague 47 et du siège 171 forment une partie de ce logement à travers lesquels l'implant 7 peut être introduit et sorti par rapport au corps 10.

Préférentiellement, le diamètre interne de la bague 47, qui est légèrement supérieur au diamètre externe de l'implant, est un peu inférieur à celui du siège 171 (et de la jupe 173 lorsqu'elle est présente comme détaillé ci-après) pour limiter le risque de contact de l'implant 7 avec le matériau du corps du dispositif.

Dans les modes de réalisation des figures 1 à 11, et en position fermée du capot 20, une partie de l'implant 7 s'étend ainsi en dessous du siège 171 de réception de la bague 47 en étant visible pour un opérateur en vue de face du corps, en particulier du fait que la face avant du corps (opposée à la paroi de fond) est ouverte. L'implant 7 est en contact avec la pièce 8 qui est reçue en partie dans la partie supérieure du coulisseau 3.

Dans les modes de réalisation des figures 12 à 15, et en position fermée du capot 20, la partie de l'implant 7 qui s'étend en dessous du siège de réception de la bague 47 est entourée par la jupe 173 à l'intérieur de laquelle est engagée l'extrémité supérieure du coulisseau 3 qui forme un piston.

La partie supérieure 17 du corps 10 présente ainsi par l'intermédiaire de la bague 47 et du passage traversant du siège 171, une ouverture, dite ouverture d'insertion/libération de l'implant 7 (dont la partie supérieure peut être scellée dans l'attente de l'ouverture du capot). Comme rappelé ci-avant, cette ouverture permet aussi la sortie de la vis 8, et le coulisseau 3 peut être déplacé pour si besoin rapproché la vis 8 de cette ouverture de sortie.

L'axe d'insertion/libération ou axe de sortie A1 de l'implant 7 correspond dans l'exemple illustré aux figures, à l'axe longitudinal du corps 10 du dispositif.

Autrement dit, lorsque l'opérateur tient le dispositif face à lui, c'est-à-dire avec le capot 20 pivoté orienté vers lui, l'ouverture d'insertion/libération présentée par le corps du dispositif est située sensiblement du côté de l'opérateur.

Le fait qu'une extrémité de l'objet s'étende dans le corps et que l'autre extrémité soit coiffée par le capot en position fermée dudit capot, permet un bon maintien et une bonne protection de l'objet.

### Capot

Dans l'exemple illustré aux figures, le capot 20 comprend une forme générale en U renversé.

Le capot 20 présente ainsi deux bras 21 reliés entre eux par une partie de liaison 22 apte à être saisie par une main d'un opérateur. Le capot 20 est couplé au corps 10 par ses bras 21.

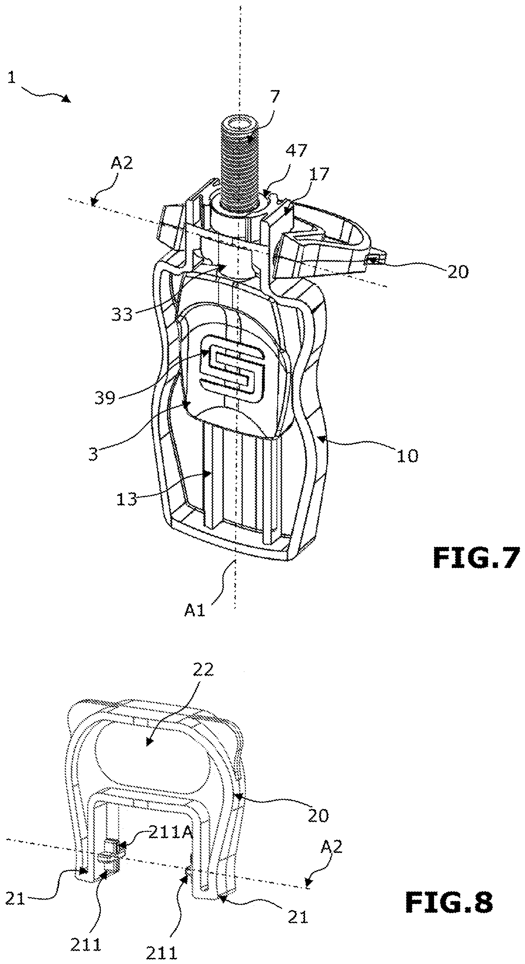

Comme illustré plus particulièrement aux figures 8 et 3, chaque bras 21 est muni d'un élément 211, par exemple un élément mâle en forme de croisillons, apte à coopérer avec un élément 121 femelle correspondant du corps 10, pour permettre un pivotement du capot 20 par rapport au corps 10.

Le pivotement du capot 20 s'effectue autour d'un axe A2 orthogonal à l'axe de sortie A1 de l'objet par rapport au corps 10. En particulier dans l'exemple illustré aux figures, l'axe A2 de pivotement est parallèle au plan moyen du corps (ou encore au plan moyen de coulissement du coulisseau 3).

Les éléments de pivotement 211,121 ménagés sur le capot 20 et sur le corps 10 permettent un pivotement du capot 20 par rapport au corps 10, de part et d'autre de l'axe de sortie A1. Autrement dit, le capot 20 peut être pivoté d'un côté ou de l'autre du plan moyen du corps 10 qui passe par cet axe de sortie A1.

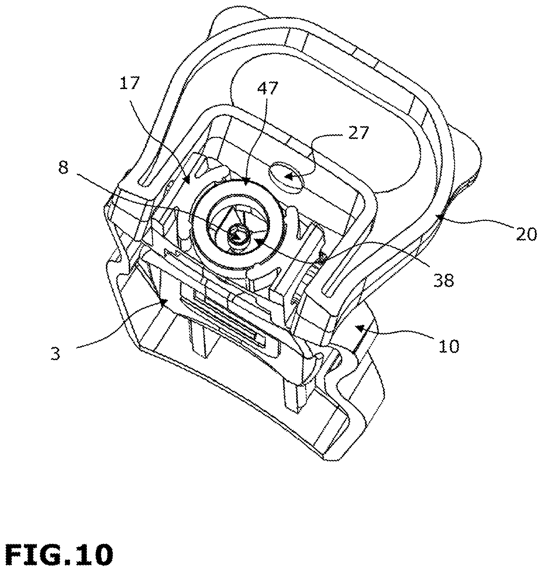

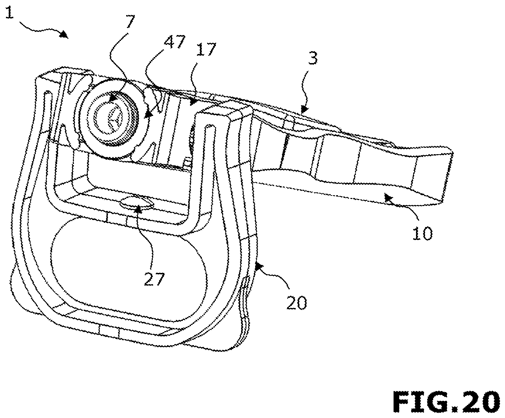

La partie de liaison 22 du capot 20 présente un bord intérieur qui, en position de fermeture du capot 20, se situe en regard du passage de sortie 107 du corps 10. Comme illustré plus particulièrement à la figure 10 et à la figure 20, ledit bord intérieur du capot 20 est muni d'un relief saillant 27 en direction dudit passage de sortie, de préférence à courbure convexe, par exemple en forme de demi-sphère, pour venir coopérer avec l'extrémité creuse de l'implant 7, et assurer ainsi le centrage axial de l'implant dans le passage de sortie.

### Système d'indexation des positions du capot

Les positions de fermeture et d'ouverture du capot 20 sont des positions prédéfinies (discrètes) stables. Autrement dit, l'opérateur doit exercer un effort au-delà d'une valeur seuil pour faire pivoter le capot 20 d'une position à une autre.

En position de fermeture, le capot 20 permet d'empêcher ou gêner l'accès de l'opérateur à l'objet afin de prévenir une sortie ou dégradation intempestive de l'objet.

Une partie du mécanisme de pivotement entre le capot 20 et le corps 10 est ménagée sur la partie supérieure 17 du corps 10 du dispositif. En particulier, dans l'exemple illustré aux figures, ladite partie de mécanisme de pivotement est située à proximité du siège 171 de réception, et comprend deux éléments 112 répartis de part et d'autre dudit siège 171 sur les bords latéraux supérieurs dudit corps 10.

Dans l'exemple illustré aux figures, le mécanisme de pivotement présente un système d'indexation de position qui permet de pivoter le capot 20 dans des positions où il est maintenu (stable), de préférence aussi bien pour le pivotement du capot 20 dans un sens que dans l'autre.

Le système d'indexation du capot 20 offre aussi une position d'indexation correspondant à la position fermée du capot 20, ce qui permet de maintenir le capot 20 dans cette position fermée, tant que l'opérateur ne force pas le passage du capot 20 dans une autre position indexée, correspondant à une position d'ouverture du capot, dans un sens ou dans l'autre.

Ces positions stabilisées du capot sont obtenues dans l'exemple illustré aux figures, par au moins un bras des croisillons qui est muni d'une dent 211A (visible à la figure 8) apte à s'engager dans les creux 121A d'un élément 121 ménagé sur le corps 10 (voir figure 3), en fonction de la position angulaire du capot 20 par rapport au corps 10. A cet effet l'élément 121 présente trois creux écartés angulairement les uns des autres selon les positions stables souhaitées de fermeture, d'ouverture d'un côté et d'ouverture de l'autre côté.

Ainsi, en tournant le capot 20 par rapport au corps 10, le capot 20 est amené dans une nouvelle position stable indexée par coopération de la dent 121A de chaque croisillon 121 avec l'un des creux122A de l'élément 121 correspondant du corps 10.

Les éléments 211, 121 de pivotement et d'indexation en position peuvent être répartis indifféremment sur le capot 20 et le corps 10. Ainsi dans l'exemple illustré aux figures, les croissillons sont portés par les bras 21 du capot 20 tandis que les logements complémentaires de ces croisillons sont ménagés sur le corps 10. Mais une répartition des croisillons sur le corps et des logements complémentaires sur les bras du capot peut aussi être prévue.

La partie de liaison 22 du capot 20 présente une partie plate apte à porter une inscription, telle qu'un logo, permettant de personnaliser ledit capot.

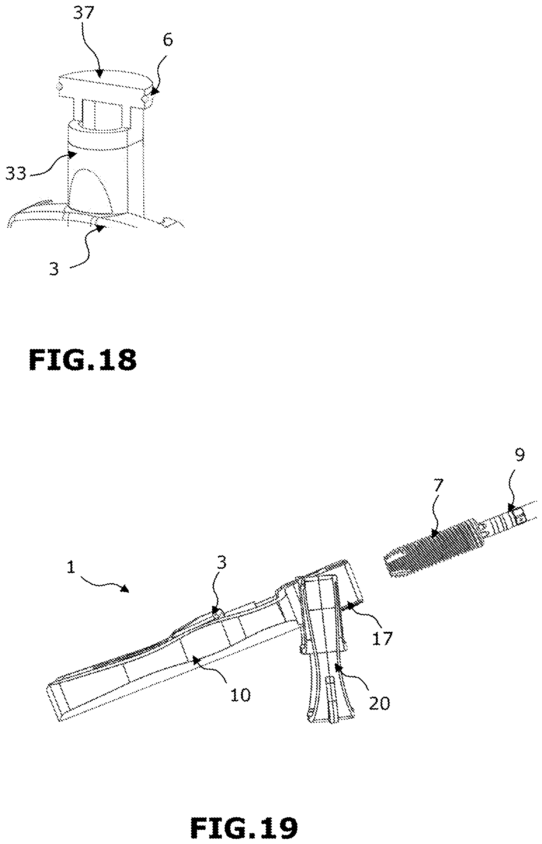

L'opérateur peut ainsi pivoter le capot 20 en une position d'ouverture stable prédéfinie, de préférence aussi bien d'un côté ou de l'autre du corps du dispositif. Le capot 20 ainsi pivoté forme un pied par l'intermédiaire duquel le dispositif peut reposer sur une surface de pose, tel qu'une table, de sorte que le corps du dispositif est incliné par rapport à la surface de pose comme illustré à la figure 19. Avantageusement, le corps du dispositif est incliné par rapport à la surface de pose d'un angle de l'ordre de 30°. On comprend que cet angle dépend de la longueur du corps et des positions stables prédéfinies de pivotement du capot 20 par rapport au corps 10 du dispositif. Préférentiellement, le capot forme en position d'ouverture un angle de 90° par rapport au corps 10.

La partie supérieure 17 du corps 10 par laquelle l'implant 7 est destiné à être sorti est ainsi écartée de la surface de pose du dispositif.

### Coulisseau

La mobilité du coulisseau permet comme rappelé ci-dessus de régler la position du coulisseau à la position et donc à la longueur de l'objet formé de l'ensemble des pièces 7, 8 de sorte que les pièces 7 et 8 peuvent amenée l'une en contact de l'autre par remontée du coulisseau jusqu'à la mise en contact de la pièce supérieure 7 avec le capot 20 (en l'absence d'élément de scellage entre le capot et l'ouverture de sortie du corps).

La configuration empêchant un recul du coulisseau permet de s'assurer que l'objet 7, 8 est prisonnier entre le coulisseau 3 et le capot 20 tant que celui-ci n'est pas amené en position d'ouverture, ce qui évite que tout ou partie de l'objet ne tombe au fond du corps 10 ou dans une zone du corps 10 non prévue pour le logement de l'objet.

Le coulisseau 3, à l'état remonté contre les pièces 7, 8, forme une butée basse tandis que le capot (ou un élément de scellage intermédiaire comme décrit ci-après) forme une butée haute.

En position d'ouverture du capot, le coulisseau 3 permet aussi de pousser l'objet formé de l'ensemble des pièces 7, 8 le long de l'axe A1 dans le sens d'un rapprochement de l'extrémité de la pièce 8 qui est en contact avec le coulisseau 3, vers la partie supérieure 17 du corps, en vue de l'extraction de la vis implant 7 et de la vis 8 de cicatrisation hors du corps 10.

Dans cette position de sortie du coulisseau 3 et à l'état extrait de la pièce 7, la pièce 8 de l'objet se trouve à proximité et en regard du passage traversant de la bague 47, ce qui permet à l'opérateur de saisir la pièce 8 à l'aide d'un outil 9' en passant ledit outil à travers 47.

Selon certains modes de réalisation le capot 20, le corps 10 et le coulisseau 3 sont configurés de telle sorte que la longueur de l'implant, c'est-à-dire au moins ses extrémités, reste visible. La longueur de l'implant peut ainsi rester visible au moins pour l'opérateur qui regarde le dispositif en vue de face, c'est-à-dire du côté du coulisseau, en configuration normale de saisie du dispositif.

Le coulisseau est muni d'une zone 39 de positionnement d'un doigt, encore appelée empreinte, par exemple comprenant des irrégularités de surface en creux ou en relief, telles que des nervures ou stries, facilitant l'entrainement en déplacement à coulissement du coulisseau 3 par l'opérateur.

### Partie supérieure du coulisseau

Le coulisseau 3 présente une partie supérieure 33 qui correspond à la partie d'extrémité du coulisseau 3 orientée vers le passage de sortie 107 du corps 10.

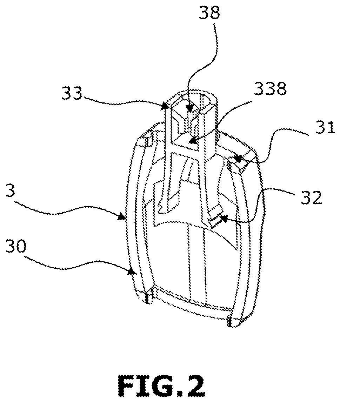

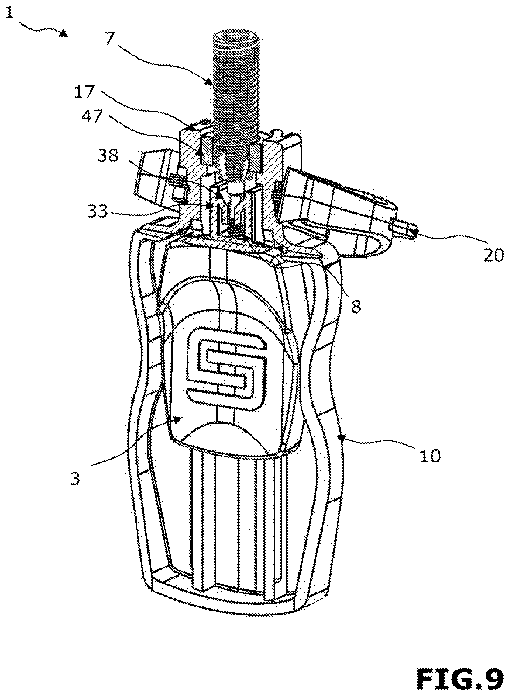

Comme visible plus particulièrement à la figure 2 et à la figure 9, la partie supérieure 33 comprend une partie 38 de réception qui permet de recevoir une partie de la pièce 8.

Dans l'exemple illustré aux figures 1 à 11, la partie 38 de réception présente une forme d'entonnoir et l'extrémité de cette pièce 8 opposée à l'implant est située en regard et/ou en contact avec une partie 338 du coulisseau formant butée basse pour la pièce 8. La partie 338 permet ainsi d'arrêter la pièce 8 de manière à limiter ou contrôler son insertion dans le coulisseau 3 pour conserver une partie de la pièce 8 qui s'étend en saillie de du coulisseau, en particulier en saillie de l'entonnoir 38 pour venir en contact avec la pièce 7.

### Exemple d'utilisation pour les modes de réalisation des figures 1 à 11

Dans l'exemple illustré aux figures 1 à 11, lors de la production du dispositif avec introduction de la vis-implant 7 et de la vis de cicatrisation 8 dans le corps du dispositif, la vis de cicatrisation 8 peut être introduite dans le logement correspondant 38 du coulisseau 3, sans avoir à passer par la bague 47, par exemple en étant introduite depuis la face ouverte du corps du dispositif dans l'extrémité haute du coulisseau 3 qui est alors en position basse, c'est-à-dire à proximité de l'extrémité basse du corps 10.

Puis la vis implant 7 peut être introduite dans la partie supérieure du corps 10 par le passage de sortie 107 correspondant (voir figure 3), dans une position où l'extrémité supérieure de la vis est située à proximité de l'extrémité supérieure du corps, et où la bague 47 entoure ladite vis implant (Autrement dit, la vis implant ne s'étend pas entièrement au-dessous de la bague).

Le capot 20 peut être fermé pour former une butée haute vis-à-vis de la vis implant 7. La vis implant 7 et la vis de cicatrisation 8 sont alors mises en contact l'une de l'autre par remontée du coulisseau 3 en direction de la vis implant 7, ce qui entraine aussi la mise en butée de la vis implant 7 contre le capot 20.

Lors de l'opération de sortie de l'implant 7, l'implant 7 est extrait sans entrainer la vis 8 de cicatrisation. Comme illustré à la figure 10, la vis 8 de cicatrisation reste supportée par la partie d'extrémité 33 du coulisseau 3 et peut être extraite du corps une fois la vis implant 7 extraite. Autrement dit, dans les exemples illustrés aux figures, il n'y a pas de couplage mécanique particulier entre la vis implant 7 et la vis 8 de cicatrisation.

On obtient ainsi un dispositif correspondant à celui illustré par exemple à la figure 1.

Lorsque l'opérateur souhaite extraire les pièces 7,8 du dispositif 1, il peut alors ouvrir le capot 20 pour accéder à l'implant 7 par la sortie du corps 10, en faisant sortir l'implant 7 par exemple avec l'outil 9 qui est adapté à être couplé à l'implant, par exemple par couplage d'une extrémité mâle de l'outil avec l'extrémité creuse de l'implant 7.

Comme illustré aux figures 7, 9, l'opérateur peut aussi pousser le coulisseau 3 pour faire sortir l'implant 7 et le saisir. A noter qu'une telle sortie de l'implant 7 par poussée du coulisseau 3 permet de saisir l'implant, manuellement ou à l'aide d'un outil adapté, par son pourtour extérieur.

Comme illustré à la figure 10, la poussée sur le coulisseau 3 permet d'amener la pièce 8 au voisinage de la sortie du corps 10. En particulier, l'opérateur peut alors passer un outil 9' adapté à travers la bague 47 pour aller chercher la pièce 8 et l'extraire du coulisseau et du corps.

A noter que la position haute poussée du coulisseau 3 facilite l'extraction de la vis de cicatrisation car sinon la collerette 90' de l'outil 9' pourrait taper contre la bague 47 ou le siège 171 de la bague en tentant de ressortir l'outil 9' entré trop profondément dans le corps 10 du dispositif 1.

### Exemple d'utilisation pour les modes de réalisation des figures 12 à 15

Dans les exemples des figures 12 à 15, le piston 37 du coulisseau 3 est introduit dans la partie inférieure de la jupe 173. On peut prévoir que le piston présente aussi une forme adaptée, par exemple comme la forme de la partie supérieure 33 avec entonnoir 38 et la butée basse 338 du mode de réalisation de la figure 2, de manière à recevoir une pièce, telle que la pièce 8 présentée dans les modes de réalisation des figures 1 à 11, tout en conservant une fonction de piston, c'est-à-dire avec un pourtour périphérique adapté à être déplacé de manière étanche à l'intérieur de la jupe 173.

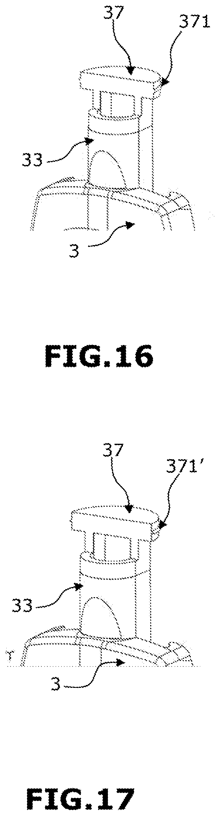

En particulier, on peut prévoir comme illustré aux figures 16 à 18, que l'étanchéité soit assurée par un élément périphérique formé par une partie périphérique du chant de la tête de piston à section semi-circulaire 371 (Figure 16), à section en quart de rond 371' (avec courbure orientée vers l'intérieur le haut) (Figure 17) ou formé par un joint 6 (Figure 18), de préférence à section circulaire, logé dans une gorge périphérique ménagée dans le chant de la tête de piston.

Une rainure ou gorge axiale peut être ménagée dans la bague pour faciliter l'expulsion de l'air lors de l'introduction du piston 37 dans la jupe 173.

L'implant 7 est introduit dans la jupe 173 par la sortie du corps 10 en passant par la bague 47. Avantageusement, une partie de l'implant reste entourée par la bague 47.

Selon un aspect particulier, la chambre délimitée au moins par la jupe 173 et le piston 37 est rempli de liquide, par exemple une solution de chlorure de sodium pouvant permettre de conserver un état hautement hydrophile à la surface de l'implant, ou un tensio-actif permettant de maintenir un état de surface actif et non modifié par contact avec l'air, ou encore un élément de dopage osteo-promoteur, par exemple une solution de BMP (BMP pour Bone Morphogenetic Protein en anglais), ou encore un élément de désinfection / prévention bactéricide (solution d'ion Argent ou autres....), dans lequel baigne alors l'implant 7. La sortie du corps 10 est dans ce cas scellée pour contenir le liquide et le capot peut être amené en position fermée en regard de la sortie.

L'opérateur qui souhaite extraire l'implant 7 et la pièce 8 lorsqu'elle est présente, peut alors ouvrir le capot, et, de préférence simultanément, retirer l'élément de scellage 273, pour pouvoir accéder à la vis. L'implant 7 (et la pièce 8 lorsqu'elle présente) peut alors être retiré comme expliqué ci-avant à l'aide d'un outil 9 adapté avec ou sans poussée du coulisseau 3.

Une poussée du coulisseau 3 fait aussi sortir le liquide dans lequel baigne l'implant, à la manière d'une seringue dont le piston serait le piston 37.

Préférentiellement, l'élément de scellage 273 utilisé est attaché au capot 20, de sorte que l'ouverture du capot 20 entraine le retrait de l'élément de scellage 273 par rapport à la sortie du corps du dispositif. Ainsi, la représentation du capot en cours d'ouverture à la figure 12, laisse apparaitre l'extrémité haute de l'implant 7 qui est entouré par la bague 47 et la jupe 173, l'élément de scellage 273 de l'extrémité haute du logement de l'implant étant défait ou retiré par rapport au corps 10 en même temps que le capot 20 s'ouvre.

En variante, on peut prévoir que l'élément de scellage ne soit pas lié au capot et qu'il soit retiré manuellement pour libérer la sortie du corps 10 et ainsi ouvrir la chambre qui contient l'implant 7.

Une référence dans toute la spécification à « un mode de réalisation » signifie qu'une fonctionnalité, une structure, ou une caractéristique particulière décrite en relation avec un mode de réalisation est incluse dans au moins un mode de réalisation de la présente invention. Ainsi, l'apparition de l'expression « dans un mode de réalisation » à divers emplacements dans toute la spécification ne fait pas nécessairement référence au même mode de réalisation. En outre, les fonctionnalités, les structures, ou les caractéristiques particulières peuvent être combinées de n'importe quelle manière appropriée dans un ou plusieurs modes de réalisation.

L'invention n'est pas limitée aux modes de réalisation illustrés dans les dessins.

De plus, le terme « comprenant » n'exclut pas d'autres éléments ou étapes. En outre, des caractéristiques ou étapes qui ont été décrites en référence à l'un des modes de réalisation exposés ci-dessus peuvent également être utilisées en combinaison avec d'autres caractéristiques ou étapes d'autres modes de réalisation exposés ci-dessus.

## Revendications

1. Dispositif (1) pour le maintien et la libération d'un objet médical (7, 8), le dispositif comprenant un corps (10) principal présentant un logement pour loger au moins partiellement ledit objet médical (7,8) et une sortie (107) pour permettre la sortie d'au moins une partie de l'objet médical (7,8), **caractérisé en ce que** ledit dispositif (1) pour le maintien et la libération de l'objet médical (7,8) comprend aussi :
- un capot (20) monté mobile entre une position de fermeture empêchant l'accès à ladite sortie (107) et une position d'ouverture libérant l'accès à ladite sortie (107),
- un dispositif de butée (3) configuré pour être déplaçable en différentes positions par rapport au corps (10) principal en direction de la sortie (107) dudit corps (10) principal, mais bloqué en sens inverse, pour permettre d'amener le dispositif de butée (3) en contact ou à proximité de l'objet médical (7,8) tout en empêchant un recul du dispositif de butée (3).

2. Dispositif selon la revendication 1, dans lequel un système de crémaillère (132, 32) est configuré pour permettre ledit déplacement du dispositif de butée (3) en direction de la sortie (107) dudit corps (10) principal, avec blocage en sens inverse.

3. Dispositif selon la revendication 1 ou 2, dans lequel le dispositif de butée (3) est configuré pour être déplaçable par rapport au corps (10) principal sur une distance permettant d'amener au moins une partie de l'objet médical (7,8) en saillie du corps (10) principal.

4. Dispositif selon l'une des revendications précédentes, dans lequel le dispositif de butée (3) présente un logement (38) apte à recevoir une partie (8) de l'objet médical (7, 8).

5. Dispositif selon l'une des revendications précédentes, dans lequel le logement du corps (10) principal comprend une bague (47) à travers laquelle au moins une partie de l'objet médical (7,8) est apte à s'étendre, ladite bague (47) étant réalisée en un matériau différent de celui du corps (10) principal.

6. Dispositif (1) selon la revendication 5, dans lequel le logement de l'objet médical (7, 8) ménagé dans le corps principal (10) présente une partie entre la bague (47) et le dispositif de butée (3), qui reste ouverte sur l'intérieur du corps (10) principal.

7. Dispositif (1) selon la revendication 5, dans lequel le logement de l'objet médical (7, 8) comprend une jupe (173) qui s'étend au moins sous la bague (47), et le dispositif de butée (3) comprend une partie d'extrémité (37) formant piston apte à s'étendre de manière étanche à l'intérieur de la jupe (173).

8. Dispositif (1) selon la revendication 7, dans lequel la jupe (173) contient un fluide, de préférence un liquide, ledit objet médical (7, 8) étant logé dans une chambre définie au moins par ladite jupe (173) et ladite bague (47), ladite chambre étant fermée en partie basse par le piston du dispositif de butée (3) et en partie haute par un système de scellage (273).

9. Dispositif (1) selon l'une des revendications précédentes, dans lequel la sortie (107) du corps (10) principal est scellée par un système de scellage (273) retirable.

10. Dispositif (1) selon la revendication 9, dans lequel le système de scellage (273) est couplé avec le capot (20) de telle sorte que le passage du capot (20) de la position de fermeture à la position d'ouverture entraîne le retrait du système de scellage (273).

11. Dispositif selon l'une des revendications précédentes, dans lequel un système de stabilisation (121A, 211A) est configuré pour stabiliser le capot (20) en position d'ouverture et en position de fermeture.

12. Dispositif (1) selon l'une des revendications précédentes, dans lequel le capot (20) est configuré pour, en position d'ouverture, rester couplé au corps (10) principal en formant un pied permettant de disposer le corps (10) principal en appui par ce pied sur une surface, tandis que la sortie (107) dudit corps (10) principal est surélevée par rapport à cette surface.

13. Dispositif (1) selon l'une des revendications précédentes, dans lequel le capot (20) comprend un pion de centrage (27) apte à venir en contact avec une extrémité de l'objet médical (7) en position de fermeture du capot (20).

14. Dispositif (1) selon l'une des revendications précédentes, dans lequel ledit dispositif (1) comprend ledit objet médical (7, 8).

15. Dispositif selon la revendication 14, dans lequel ledit objet médical (7, 8) comprend deux pièces (7, 8) distinctes.

16. Dispositif selon la revendication 14 ou 15, dans lequel ledit objet médical (7, 8) étant de type dentaire, ledit objet médical comprend une vis-implant (7) et une vis de cicatrisation (8).

17. Procédé de production d'un dispositif (1) de maintien et de libération d'un objet médical (7, 8) conforme à l'une des revendications 14 à 16, ledit procédé comprenant les étapes suivantes :
- le capot (20) étant en position d'ouverture, introduction d'au moins une partie (7) de l'objet médical (7, 8) dans le corps (10) principal par la sortie (107) dudit corps (10) principal ;
- déplacement du dispositif de butée (3) en direction de la sortie (107) dudit corps (10) principal, pour empêcher ou limiter le déplacement de l'objet médical (7,8) dans le corps principal, le recul du dispositif de butée (3) étant empêché.

18. Procédé de production selon la revendication 17, dans lequel, ladite au moins une partie (7) de l'objet médical introduite dans le corps (10) principal par la sortie (107) étant une première pièce (7), ledit procédé comprend, avant ladite étape de déplacement du dispositif de butée (3), l'étape d'introduction dans le dispositif de butée (3) d'une deuxième pièce (8), ladite étape de déplacement du dispositif de butée (3) en direction de la sortie (107) dudit corps (10) principal amenant ladite deuxième pièce (8) en contact ou à proximité de la première pièce (7), le recul du dispositif de butée (3) étant empêché.

19. Procédé de production selon la revendication 17 ou 18, dans lequel ledit procédé comprend une étape de remplissage en fluide ou en liquide d'une chambre du corps (10) principal dans laquelle loge au moins une partie dudit objet médical (7, 8).

20. Procédé de production selon l'une des revendications 17 à19, dans lequel ledit procédé comprend une étape de scellage de la sortie (107) du corps principal.

21. Procédé de production selon l'une des revendications 17 à 20, dans lequel ledit procédé comprend une étape de fermeture du capot (20), avant ou après le déplacement du dispositif de butée (3).

22. Procédé de libération d'un objet médical (7, 8) maintenu dans un dispositif conforme à l'une des revendications 14 à 16, ledit procédé comprend les étapes suivantes :
- faire passer le capot (20) en position d'ouverture,
- extraire au moins une partie (7) de l'objet médical (7,8) par la sortie (107) du corps (10) principal à l'aide d'un outil (9) coopérant avec ladite au moins une partie (7) de l'objet médical.

23. Procédé de libération selon la revendication 22, dans lequel ledit procédé comprend les étapes de :
- pousser le dispositif de butée (3) en direction de la sortie (107) du corps principal pour amener une autre partie (8) de l'objet médical (7, 8) à proximité de la sortie (107) du corps principal, et
- extraire ladite autre partie (8) de l'objet médical (7,8) par la sortie (107) du corps principal à l'aide d'un outil (9') coopérant avec ladite autre partie (8) de l'objet (7, 8).

24. Procédé de libération d'un objet médical (7,8) maintenu dans un dispositif (1) conforme à l'une des revendications 14 à 16, ledit procédé comprenant les étapes suivantes :
- faire passer le capot (20) en position d'ouverture,
- pousser le dispositif de butée (3) pour amener une partie (7) de l'objet médical en saillie de la sortie (107) du corps principal,
- extraire ladite partie (7) de l'objet médical (7,8) qui s'étend en saillie de la sortie (107) du corps principal ;
- extraire une autre partie (8) de l'objet médical (7,8) à travers la sortie (107) du corps principal à l'aide d'un outil (9') coopérant avec ladite autre partie (8) de l'objet médical (7, 8).

## Patentansprüche

1. Vorrichtung (1) zum Halten und Freigeben eines medizinischen Objekts (7, 8), wobei die Vorrichtung einen Hauptkörper (10) umfasst, der eine Aufnahme aufweist, um mindestens teilweise das medizinische Objekt (7, 8) aufzunehmen, und einen Ausgang (107), um den Austritt mindestens eines Teils des medizinischen Objekts (7, 8) zu ermöglichen,
**dadurch gekennzeichnet, dass** die Vorrichtung (1) zum Halten und Freigeben des medizinischen Objekts (7, 8) auch Folgendes umfasst:
- eine Haube (20), die beweglich zwischen einer Verschlussposition, die den Zugang zu dem Ausgang (107) verhindert, und einer Öffnungsposition montiert ist, die den Zugang zu dem Ausgang (107) freigibt,
- eine Anschlagvorrichtung (3), die konfiguriert ist, um in verschiedene Positionen mit Bezug auf den Hauptkörper (10) in Richtung des Ausgangs (107) des Hauptkörpers (10) verschoben werden zu können, jedoch blockiert in der entgegengesetzten Richtung, um zu ermöglichen, die Anschlagvorrichtung (3) in Kontakt mit dem oder in die Nähe des medizinischen Objekt(s) (7, 8) zu bringen und gleichzeitig einen Rückzug der Anschlagvorrichtung (3) zu verhindern.

2. Vorrichtung nach Anspruch 1, wobei ein Reißverschlusssystem (132, 32) konfiguriert ist, um die Verschiebung der Anschlagvorrichtung (3) in Richtung des Ausgangs (107) des Hauptkörpers (10) mit der Blockierung in der entgegengesetzten Richtung zu ermöglichen.

3. Vorrichtung nach Anspruch 1 oder 2, wobei die Anschlagvorrichtung (3) konfiguriert ist, um mit Bezug auf den Hauptkörper (10) auf einer Distanz verschoben werden zu können, die ermöglicht, mindestens einen Teil des medizinischen Objekts (7, 8) in Vorsprung vom Hauptkörper (10) zu bringen.

4. Vorrichtung nach einem der vorhergehenden Ansprüche, wobei die Anschlagvorrichtung (3) eine Aufnahme (38) aufweist, die ausgelegt ist, um einen Teil (8) des medizinischen Objekts (7, 8) zu empfangen.

5. Vorrichtung nach einem der vorhergehenden Ansprüche, wobei die Aufnahme des Hauptkörpers (10) einen Ring (47) umfasst, durch den sich mindestens ein Teil des medizinischen Objekts (7, 8) erstrecken kann, wobei der Ring (47) aus einem Material hergestellt ist, das verschieden von demjenigen des Hauptkörpers (10) ist.

6. Vorrichtung (1) nach Anspruch 5, wobei die Aufnahme des medizinischen Objekts (7, 8), die im Hauptkörper (10) angebracht ist, einen Teil zwischen dem Ring (47) und der Anschlagvorrichtung (3) aufweist, der hin zum Inneren des Hauptkörpers (10) geöffnet bleibt.

7. Vorrichtung (1) nach Anspruch 5, wobei die Aufnahme des medizinischen Objekts (7, 8) eine Schürze (173) umfasst, die sich mindestens unter dem Ring (47) erstreckt, und die Anschlagvorrichtung (3) einen Endteil (37) umfasst, der einen Kolben bildet, der sich auf dichte Weise in das Innere der Schürze (173) erstrecken kann.

8. Vorrichtung (1) nach Anspruch 7, wobei die Schürze (173) ein Fluid enthält, vorzugsweise eine Flüssigkeit, wobei das medizinische Objekt (7, 8) in einer Kammer aufgenommen ist, die mindestens durch die Schürze (173) und den Ring (47) definiert ist, wobei die Kammer am unteren Teil durch den Kolben der Anschlagvorrichtung (3) und im oberen Teil durch ein Versiegelungssystem (273) geschlossen ist.

9. Vorrichtung (1) nach einem der vorhergehenden Ansprüche, wobei der Ausgang (107) des Hauptkörpers (10) durch ein abnehmbares Versiegelungssystem (273) versiegelt ist.

10. Vorrichtung (1) nach Anspruch 9, wobei das Versiegelungssystem (273) mit der Haube (20) derart gekoppelt ist, dass der Übergang der Haube (20) von der Verschlussposition in die Öffnungsposition den Rückzug des Versiegelungssystems (273) nach sich zieht.

11. Vorrichtung nach einem der vorhergehenden Ansprüche, wobei ein Stabilisierungssystem (121A, 211A) konfiguriert ist, um die Haube (20) in der Öffnungsposition und in der Verschlussposition zu stabilisieren.

12. Vorrichtung (1) nach einem der vorhergehenden Ansprüche, wobei die Haube (20) konfiguriert ist, um in der Öffnungsposition mit dem Hauptkörper (10) gekoppelt zu bleiben und einen Fuß zu bilden, der ermöglicht, den Hauptkörper (10) in Auflage durch diesen Fuß auf einer Fläche anzuordnen, während der Ausgang (107) des Hauptkörpers (10) mit Bezug auf diese Fläche erhöht ist.

13. Vorrichtung (1) nach einem der vorhergehenden Ansprüche, wobei die Haube (20) einen Zentrierstift (27) umfasst, der mit einem Ende des medizinischen Objekts (7) in der Verschlussposition der Haube (20) in Kontakt kommen kann.

14. Vorrichtung (1) nach einem der vorhergehenden Ansprüche, wobei die Vorrichtung (1) das medizinische Objekt (7, 8) umfasst.

15. Vorrichtung nach Anspruch 14, wobei das medizinische Objekt (7, 8) zwei verschiedene Stücke (7, 8) umfasst.

16. Vorrichtung nach Anspruch 14 oder 15, wobei, da das medizinische Objekt (7, 8) der dentalen Art ist, das medizinische Objekt ein Schraubenimplantat (7) und eine Heilungsschraube (8) umfasst.

17. Verfahren zur Herstellung einer Vorrichtung (1) zum Halten und Freigeben eines medizinischen Objekts (7, 8) nach einem der Ansprüche 14 bis 16, wobei das Verfahren die folgenden Schritte umfasst:
- wenn sich die Haube (20) in der Öffnungsposition befindet, Einführen mindestens eines Teils (7) des medizinischen Objekts (7, 8) in den Hauptkörper (10) durch den Ausgang (107) des Hauptkörpers (10);
- Verschieben der Anschlagvorrichtung (3) in Richtung des Ausgangs (107) des Hauptkörpers (10), um die Verschiebung des medizinischen Objekts (7, 8) in den Hauptkörper (10) zu verhindern oder zu begrenzen, wobei der Rückzug der Anschlagvorrichtung (3) verhindert wird.

18. Verfahren zur Herstellung nach Anspruch 17, wobei, da der mindestens eine Teil (7) des medizinischen Objekts, der in den Hauptkörper (10) durch den Ausgang (107) eingeführt ist, ein erstes Stück (7) ist, das Verfahren, vor dem Schritt des Verschiebens der Anschlagvorrichtung (3) den Schritt des Einführens in die Anschlagvorrichtung (3) eines zweiten Stücks (8) umfasst, wobei der Schritt des Verschiebens der Anschlagvorrichtung (3) in Richtung des Ausgangs (107) des Hauptkörpers (10) das zweite Stück (8) in Kontakt mit dem oder in die Nähe des ersten Stück(s) (7) bringt, wobei der Rückzug der Anschlagvorrichtung (3) verhindert wird.

19. Verfahren zur Herstellung nach Anspruch 17 oder 18, wobei das Verfahren einen Schritt des Füllens mit Fluid oder mit Flüssigkeit einer Kammer des Hauptkörpers (10) umfasst, in der mindestens ein Teil des medizinischen Objekts (7, 8) aufgenommen ist.

20. Verfahren zur Herstellung nach einem der Ansprüche 17 bis 19, wobei das Verfahren einen Schritt des Versiegelns des Ausgangs (107) des Hauptkörpers (10) umfasst.

21. Verfahren zur Herstellung nach einem der Ansprüche 17 bis 20, wobei das Verfahren einen Schritt des Schließens der Haube (20) vor oder nach dem Verschieben der Anschlagvorrichtung (3) umfasst.

22. Verfahren zum Freigeben eines medizinischen Objekts (7, 8), gehalten in einer Vorrichtung nach einem der Ansprüche 14 bis 16, wobei das Verfahren die folgenden Schritte umfasst:
- Versetzen der Haube (20) in die Öffnungsposition,
- Extrahieren mindestens eines Teils (7) des medizinischen Objekts (7, 8) durch den Ausgang (107) des Hauptkörpers (10), mit Hilfe eines Werkzeugs (9), das mit dem mindestens einen Teil (7) des medizinischen Objekts zusammenarbeitet.

23. Verfahren zum Freigeben nach Anspruch 22, wobei das Verfahren die folgenden Schritte umfasst:
- Schieben der Anschlagvorrichtung (3) in Richtung des Ausgangs (107) des Hauptkörpers, um einen anderen Teil (8) des medizinischen Objekts (7, 8) in die Nähe des Ausgangs (107) des Hauptkörpers (10) zu bringen, und
- Extrahieren des anderen Teils (8) des medizinischen Objekts (7, 8) durch den Ausgang (107) des Hauptkörpers mit Hilfe eines Werkzeugs (9'), das mit dem anderen Teil (8) des Objekts (7, 8) zusammenarbeitet.

24. Verfahren zum Freigeben eines medizinischen Objekts (7, 8), gehalten in einer Vorrichtung (1) nach einem der Ansprüche 14 bis 16, wobei das Verfahren die folgenden Schritte umfasst:
- Versetzen der Haube (20) in die Öffnungsposition,
- Schieben der Anschlagvorrichtung (3), um einen Teil (7) des medizinischen Objekts in Vorsprung vom Ausgang (107) des Hauptkörpers (10) zu bringen,
- Extrahieren des Teils (7) des medizinischen Objekts (7, 8) das sich in Vorsprung vom Ausgang (107) des Hauptkörpers (10) erstreckt;
- Extrahieren eines anderen Teils (8) des medizinischen Objekts (7, 8) über den Ausgang (107) des Hauptkörpers mit Hilfe eines Werkzeugs (9'), das mit dem anderen Teil (8) des medizinischen Objekts (7, 8) zusammenarbeitet.

## Claims

1. A device (1) for holding and releasing a medical object (7, 8), the device comprising a main body (10) having a housing for at least partially accommodating said medical object (7, 8), and an outlet (107) that allows at least a portion of the medical object (7, 8) to be extracted, **characterized in that** said device (1) for holding and releasing the medical object (7, 8) also comprises:
- a lid (20) that can be moved between a closed position preventing access to said outlet (107) and an open position providing access to said outlet (107),
- a stop device (3) designed such that it can be moved to different positions relative to the main body (10) toward the outlet (107) of said main body (10), but blocked in the opposite direction, to enable the stop device (3) to be brought into contact with or close to the medical object (7, 8) while preventing any recoil of the stop device (3).

2. The device according to claim 1, wherein a rack system (132, 32) is configured to enable said movement of the stop device (3) toward the outlet (107) of said main body (10), and to block it in the opposite direction.

3. The device according to claim 1 or 2, wherein the stop device (3) is configured such that it can be moved with respect to the main body (10) over a distance enabling at least a portion of the medical object (7, 8) to be pushed out of the main body (10).

4. The device according to one of the preceding claims, wherein the stop device (3) has a housing (38) which can accommodate a portion (8) of the medical object (7, 8).

5. The device according to one of the preceding claims, wherein the housing of the main body (10) comprises a ring (47) through which at least a portion of the medical object (7, 8) is able to extend, said ring (47) being made of a different material from that of the main body (10).

6. The device (1) according to claim 5, wherein the housing of the medical object (7, 8) arranged in the main body (10) has a portion between the ring (47) and the stop device (3), which remains open into the interior of the main body (10).

7. The device (1) according to claim 5, wherein the housing of the medical object (7, 8) comprises a skirt (173) which extends at least underneath the ring (47), and the stop device (3) comprises an end portion (37) forming a piston which can expand in an airtight manner inside the skirt (173).

8. The device (1) according to claim 7, wherein the skirt (173) contains a fluid, preferably a liquid, said medical object (7, 8) being accommodated in a chamber defined at least by said skirt (173) and said ring (47), said chamber being closed at its lower end by the piston of the stop mechanism (3) and at its upper end by a sealing system (273).

9. The device (1) according to one of the preceding claims, wherein the outlet (107) of the main body (10) is sealed by a peelable sealing system (273).

10. The device (1) according to claim 9, wherein the sealing system (273) is coupled to the lid (20) such that moving the lid (20) from the closed position to the open position leads to the removal of the sealing system (273).

11. The device according to one of the preceding claims, wherein a stabilizing system (121 A, 211 A) is configured to stabilize the lid (20) in an open position and in a closed position.

12. The device (1) according to one of the preceding claims, wherein the lid (20) is designed to remain coupled to the main body (10), in the open position, forming a stand that enables the main body (10) to be placed on a surface and supported by this stand, with the outlet (107) of said main body (110) being elevated relative to this surface.

13. The device (1) according to one of the preceding claims, wherein the lid (20) comprises a centering pin (27) which can come into contact with one end of the medical object (7) when the lid (20) is in the closed position.

14. The device (1) according to one of the preceding claims, wherein said device (1) comprises said medical object (7, 8).

15. The device according to claim 14, wherein said medical object (7, 8) comprises two separate pieces (7, 8).

16. The device according to claim 14 or 15, wherein said medical object (7, 8) is a dental object, said medical object comprising an implant screw (7) and a healing cap (8).

17. A method for producing a device (1) for holding and releasing a medical object (7, 8) according to one of claims 14 to 16, said method comprising the following steps:
- inserting at least a portion (7) of the medical object (7, 8) into the main body (10) through the outlet (107) of said main body (10) when the lid (20) is in the open position; and
- moving the stop device (3) toward the outlet (107) of said main body (10) to prevent or restrict the movement of the medical object (7, 8) within the main body, any recoil of the stop device (3) being prevented.

18. The production method according to claim 17, wherein, said at least one portion (7) of the medical object inserted into the main body (10) through the outlet (107) being a first piece (7), said method comprises, prior to said step of moving the stop device (3), the step of inserting a second piece (8) into the stop device (3), said step of moving the stop device (3) toward the outlet (107) of said main body (10) bringing said second piece (8) into contact with or close to the first piece (7), with any recoil of the stop device (3) being prevented.

19. The production method according to claim 17 or 18, wherein said method comprises filling a chamber of the main body (10) in which at least a portion of said medical object (7, 8) is accommodated with fluid or liquid.

20. The production method according to one of claims 17 to 19, wherein said method further comprises a step of sealing the outlet (107) of the main body.

21. The production method according to one of claims 17 to 20, wherein said method further comprises a step of closing the lid (20), before or after moving the stop device (3).

22. A method for releasing a medical object (7, 8) held in a device according to one of claims 14 to 16, said method comprising the following steps:
- moving the lid (20) to the open position; and
- extracting at least a portion (7) of the medical object (7, 8) through the opening (107) in the main body (10) using a tool (9) that cooperates with said at least one portion (7) of the medical object.

23. The release method according to claim 22, wherein said method further comprises the following steps:
- pressing the stop device (3) toward the outlet (107) in the main body to bring another portion (8) of the medical object close to the outlet (107) in the main body; and
- extracting said other portion (8) of the medical object (7, 8) through the outlet (107) in the main body using a tool (9') that cooperates with said other portion (8) of the object (7, 8).

24. The method for releasing a medical object (7, 8) held in a device (1) according to one of claims 14 to 16, said method comprising the following steps:
- moving the lid (20) to the open position;
- pressing the stop mechanism (3) to push a portion (7) of the medical object through the outlet (107) of the main body;
- extracting said portion (7) of the medical object (7, 8) that is protruding from the outlet (107) of the main body;
- extracting another portion (8) of the medical object (7, 8) through the outlet (107) in the main body using a tool (9') that cooperates with said other portion (8) of the medical object (7, 8).
